# EUROPEAN PATENT SPECIFICATION

(11) **EP 2 554 994 B1**
(45) Date of publication and mention of the grant of the patent: **19.10.2016**
(21) Application number: 11765758.5
(22) Date of filing: 31.03.2011
(51) Int. Cl.: G01N 33/574, G01N 33/68

(54) **SOLUBLE INTERLEUKIN-6 RECEPTOR AS BIOMARKER**
LÖSLICHER INTERLEUKIN-6 REZEPTOR ALS BIOMARKER
RÉCEPTEUR SOLUBLE D'INTERLEUKINE 6 COMME BIOMARQUEUR

(30) Priority: 31.03.2010 JP 2010083198
(43) Date of publication of application: 06.02.2013
(73) Proprietor: National Cancer Center, Tokyo 104-0045 (JP)
(72) Inventor: HONDA, Kazufumi, Tokyo 104-0045 (JP); YAMADA, Tesshi, Tokyo 104-0045 (JP); MAKUUCHI, Yosuke, Tokyo 104-0045 (JP); OSAKA, Yoshiaki, Tokyo 166-0004 (JP); HIROHASHI, Setsuo, Tokyo 106-0032 (JP)
(74) Representative: Gill Jennings & Every LLP
(86) International application number: PCT/JP2011/058259
(87) International publication number: WO 2011/125858

(56) References cited:
- WO-A2-02/099114
- HIDEAKI SHIMADA ET AL: "Clinical significance of serum vascular endothelial growth factor in esophageal squamous cell carcinoma", CANCER, vol. 92, no. 3, 1 August 2001 (2001-08-01) , pages 663-669, XP055079300, ISSN: 0008-543X, DOI: 10.1002/1097-0142(20010801)92:3<663::AID-C NCR1368>3.0.CO;2-L
- M. GOTOH ET AL: "Epidermal Growth Factor Receptor is a Possible Predictor of Sensitivity to Chemoradiotherapy in the Primary Lesion of Esophageal Squamous Cell Carcinoma", JAPANESE JOURNAL OF CLINICAL ONCOLOGY, vol. 37, no. 9, 27 August 2007 (2007-08-27), pages 652-657, XP055079301, ISSN: 0368-2811, DOI: 10.1093/jjco/hym089
- I SNITCOVSKY ET AL: "Plasma Osteopontin Levels in Patients With Head and Neck Cancer Undergoing Chemoradiotherapy", ARCHIVES OF OTOLARYNGOLOGY - HEAD AND NECK SURGERY, vol. 135, no. 8, 17 August 2009 (2009-08-17), page 807, XP055079303, ISSN: 0886-4470, DOI: 10.1001/archoto.2009.103
- W. A. MICHAUD ET AL: "Bcl-2 Blocks Cisplatin-Induced Apoptosis and Predicts Poor Outcome Following Chemoradiation Treatment in Advanced Oropharyngeal Squamous Cell Carcinoma", CLINICAL CANCER RESEARCH, vol. 15, no. 5, 17 February 2009 (2009-02-17), pages 1645-1654, XP055079306, ISSN: 1078-0432, DOI: 10.1158/1078-0432.CCR-08-2581
- C. ALLEN ET AL: "Nuclear Factor- B-Related Serum Factors as Longitudinal Biomarkers of Response and Survival in Advanced Oropharyngeal Carcinoma", CLINICAL CANCER RESEARCH, vol. 13, no. 11, 1 June 2007 (2007-06-01), pages 3182-3190, XP055026104, ISSN: 1078-0432, DOI: 10.1158/1078-0432.CCR-06-3047
- B. M. EROVIC ET AL: "Mcl-1, Vascular Endothelial Growth Factor-R2, and 14-3-3 Expression Might Predict Primary Response against Radiotherapy and Chemotherapy in Patients with Locally Advanced Squamous Cell Carcinomas of the Head and Neck", CLINICAL CANCER RESEARCH, vol. 11, no. 24, 15 December 2005 (2005-12-15), pages 8632-8636, XP055079307, ISSN: 1078-0432, DOI: 10.1158/1078-0432.CCR-05-1170
- SHARIAT S F ET AL: "PRE-TREATMENT BIOMARKER LEVELS IMPROVE THE ACCURACY OF POST-PROSTATECTOMY NOMOGRAM FOR PREDICTION OF BIOCHEMICAL RECURRENCE", JOURNAL OF UROLOGY, LIPPINCOTT WILLIAMS & WILKINS, BALTIMORE, MD, US, vol. 181, no. 4, 1 April 2009 (2009-04-01) , page 813, XP026010316, ISSN: 0022-5347, DOI: 10.1016/S0022-5347(09)62267-9 [retrieved on 2009-03-18]
- YI-FEN WANG ET AL: "Clinical significance of interleukin-6 and interleukin-6 receptor expressions in oral squamous cell carcinoma", HEAD & NECK, vol. 24, no. 9, 1 September 2002 (2002-09-01), pages 850-858, XP055079296, ISSN: 1043-3074, DOI: 10.1002/hed.10145
- TAKEHARU KANAZAWA ET AL: "Interleukin-6 directly influences proliferation and invasion potential of head and neck cancer cells", EUROPEAN ARCHIVES OF OTO-RHINO-LARYNGOLOGY ; AND HEAD & NECK, SPRINGER, BERLIN, DE, vol. 264, no. 7, 20 February 2007 (2007-02-20), pages 815-821, XP019516772, ISSN: 1434-4726, DOI: 10.1007/S00405-007-0264-6
- SATORU SHINRIKI ET AL: "Humanized Anti-Interleukin-6 Receptor Antibody Suppresses Tumor Angiogenesis and In vivo Growth of Human Oral Squamous Cell Carcinoma", CLINICAL CANCER RESEARCH, THE AMERICAN ASSOCIATION FOR CANCER RESEARCH, US, vol. 15, no. 17, 1 September 2009 (2009-09-01), pages 5426-5434, XP002628465, ISSN: 1078-0432, DOI: 10.1158/1078-0432.CCR-09-0287 [retrieved on 2009-08-25]
- T. L. LEE ET AL: "Epigenetic modification of SOCS-1 differentially regulates STAT3 activation in response to interleukin-6 receptor and epidermal growth factor receptor signaling through JAK and/or MEK in head and neck squamous cell carcinomas", MOLECULAR CANCER THERAPEUTICS, vol. 5, no. 1, 1 January 2006 (2006-01-01) , pages 8-19, XP055079299, ISSN: 1535-7163, DOI: 10.1158/1535-7163.MCT-05-0069
- JABLONSKA EWA ET AL: "TNF-alpha, IL-6 and their soluble receptor serum levels and secretion by neutrophils in cancer patients", ARCHIVUM IMMUNOLOGIAE ET THERAPIAE EXPERIMENTALIS, BIRKHAEUSER VERLAG AG, CH, vol. 49, no. 1, 1 January 2001 (2001-01-01), pages 63-69, XP009088684, ISSN: 0004-069X
- YOSUKE MAKUUCHI ET AL: "Soluble interleukin-6 receptor is a serum biomarker for the response of esophageal carcinoma to neoadjuvant chemoradiotherapy", CANCER SCIENCE, vol. 104, no. 8, 13 August 2013 (2013-08-13), pages 1045-1051, XP055079195, ISSN: 1347-9032, DOI: 10.1111/cas.12187
- BAYER CHRISTINE ET AL.: 'PAI-1 levels predict response to fractionated irradiation in 10 human squamous cell carcinoma lines of the head and neck' RADIOTHERAPY AND ONCOLOGY vol. 86, no. 3, 2008, pages 361 - 368, XP022519077
- CHIN DAVID ET AL.: 'Novel markers for poor prognosis in head and neck cancer' INT J CANCER vol. 113, no. 5, 2005, pages 789 - 797, XP008097963
- MASAHIRO SAKAMOTO ET AL.: 'The effect of clarithromycin to prolong the survival time of patients with unresectable non-small lung cancer' JAPANESE JOURNAL OF CHEMOTHERAPY vol. 48, no. 2, 2000, pages 112 - 116, XP008161269
- ARIGA, H ET AL.: 'Potential biomarkers of a complete response and local control for definitive chemoradiotherapy in resectable esophageal squamous cell carcinoma' EJC SUPPLEMENTS vol. 7, no. 2, 2009, page 160, XP026689353
- CHIAKI ARAI ET AL.: 'Relationship between Activation of Blood Coagulation-Fibrinolysis System by Pre-operative Radiotherapy and Molecular Markers in Patients with Oral Squamous Cell Carcinoma' JAPANESE JOURNAL OF ORAL DIAGNOSIS/ORAL MEDICINE vol. 19, no. 2, 2006, pages 200 - 204, XP008161278
- TOYOJI SATO ET AL.: 'IAP no Rinsho Oyo' THE JAPANESE JOURNAL OF CLINICAL PATHOLOGY 1988, pages 101 - 107, XP008161275
- OKAMURA SHINICHI ET AL.: 'The influence of stromal inflammatory reaction on the chemoradiation responses for advanced esophageal cancer' PROCEEDINGS OF THE JAPANESE CANCER ASSOCIATION vol. 68TH, 2009, page 61, XP008161285
- KUHN D J ET AL.: 'Overexpression of Interleukin-2 Receptor a in a Human Squamous Cell Carcinoma of the Head and Neck Cell Line Is Associated With Increased Proliferation, Drug Resistance, and Transforming Ability' J CELL BIOCHEM vol. 89, no. 4, 2003, pages 824 - 836, XP008161256

## Description

### Technical field

The present invention relates to biomarkers for determining whether or not chemoradiotherapy is applicable to a patient with

### Background art

Neoadjuvant chemoradiotherapy in patients with adenocarcinoma or squamous cell carcinoma has been reported to improve survival rate in the patients compared with surgery alone (see, for example, Thomas N. et al., New England Journal of Medicine, 1996 Aug, 15: 462-467 and Val Gebski et al., Lancet Oncol., 2007 Mar, 8(3): 226-234). It is, however, known that some patients have a good response to chemoradiotherapy for cancer but others not. Discrimination of these patients before initiation of the treatment allows better choice of therapy suitable for each patient (see, for example, Shimada et al., Cancer, 2001, 92(3): 663-669; Gotoh et al., Jpn. J. Clin. Oncol., 2007, 37(9): 652-657; Snitcovsky et al., Arch. Otolaryngol. Head Neck Surg., 2009, 135(8): 807-811; Michaud et al., Clin. Cancer Res., 2009, 15(5): 1645-1654; Allen et al., Clin. Cancer Res., 2007, 13(11): 3182-3190; Erovic et al., Clin. Cancer Res., 2005, 11(24): 8632-8636).

Therefore, an object of the present invention is to provide biomarkers for predicting response to chemoradiotherapy for squamous cell carcinoma and markers for predicting prognosis of a patient with squamous cell carcinoma who has received chemoradiotherapy.

### Disclosure of the invention

More specifically, there is the use of a biomarker for predicting response to chemoradiotherapy for squamous cell carcinoma, the biomarker being soluble interleukin-6 receptor.

According to a further aspect of the invention, there is the use of a soluble interleukin-6 receptor for predicting prognosis of a patient with squamous cell carcinoma, the patient having received chemoradiotherapy.

In the aforementioned use of the biomarker for predicting response to chemoradiotherapy and the biomarker for predicting prognosis of a patient with squamous cell carcinoma, the patient having received chemoradiotherapy, the squamous cell carcinoma is preferably head and neck squamous cell carcinoma or esophageal squamous cell carcinoma.

In addition, in the use of the marker for predicting prognosis of a patient with squamous cell carcinoma, the patient having received chemoradiotherapy, the chemoradiotherapy is more preferably preoperative chemoradiotherapy.

A method for measuring a biomarker according to the present invention comprising measuring the concentration of a soluble interleukin-6 receptor in the blood obtained before treatment with chemoradiotherapy.

In the method of measuring the concentration of the biomarker, the blood has been preferably obtained from a patient with squamous cell carcinoma. In addition, it is more preferable that the concentration of the biomarker is measured by using an antibody specific to the biomarker. It is most preferable that the squamous cell carcinoma is head and neck squamous cell carcinoma or esophageal squamous cell carcinoma.

### Brief description of the drawings

Fig. 1 is a graph showing survival rates of patients for up to 9 years from the beginning of treatment in a group of patients with esophageal squamous cell carcinoma with grade 3 effects of preoperative chemoradiotherapy and a group of patients with esophageal squamous cell carcinoma with grade 1 or 2 effects, in an example of the present invention;
Fig. 2 shows plots of blood concentration of a soluble interleukin-6 receptor (sIL6R) measured using a fluorescent bead-based array system in each of groups of patients with the grade 1, 2, and 3 effects of preoperative chemoradiotherapy in an example of the present invention;
Fig. 3 is a box plot showing blood concentration of a soluble interleukin-6 receptor (sIL6R) measured using sandwich ELISA in each of groups of patients with the grade 1, 2, and 3 effects of preoperative chemoradiotherapy in an example of the present invention;
Fig. 4 is a graph showing survival rates from the beginning of treatment in patients with esophageal squamous cell carcinoma divided into groups on the basis of the threshold of 30 ng/ml of the blood sIL6R concentration measured using a fluorescent bead-based array system in an example of the present invention; and
Fig. 5 is a graph showing survival rates from the beginning
of treatment in patients with esophageal squamous cell carcinoma divided into groups on the basis of a threshold of 30 ng/ml of the blood sIL6R concentration measured using sandwich ELISA in an example of the present invention.

### Mode for carrying out the invention

Embodiments of the present invention that were completed based on the aforementioned findings are described below in detail in reference to Examples.

Unless otherwise noted in embodiments and examples, all procedures used are as described in standard protocols such as J. Sambrook, E. F. Fritsch & T. Maniatis (Ed.), Molecular cloning, a laboratory manual (3rd edition), Cold Spring Harbor Press, Cold Spring Harbor, New York (2001); F. M. Ausubel, R. Brent, R. E. Kingston, D. D. Moore, J. G. Seidman, J. A. Smith, K. Struhl (Ed.), Current Protocols in Molecular Biology, John Wiley & Sons Ltd., with or without modifications or changes. In addition, unless otherwise noted, a commercial reagent kit or a measurement instrument, if any, is used as described according to protocols attached thereto.

The above and further objects, features, advantages, and ideas of the present invention are apparent to those skilled in the art from consideration of the detailed description of this specification. Furthermore, those skilled in the art can easily reproduce the present invention from these descriptions. The mode(s) and specific example(s) described below represent a preferable embodiment of the present invention, which is given for the purpose of illustration or description. The present invention is not limited thereto. It is obvious to those skilled in the art that various modifications may be made according to the descriptions of the present specification without departing from the spirit and scope of the present invention disclosed herein.

### == Biomarkers ==

In this specification, a biomarker for predicting response to chemoradiotherapy (also referred to as a predictive marker of response) for cancer comprises a biomarker (marker of response) for identifying patients with cancer who will respond to chemoradiotherapy (responder group), and a biomarker (marker of non-response) for identifying patients with cancer who will not respond to chemoradiotherapy (non-responder group). In addition, a biomarker for predicting prognosis of patients with squamous cell carcinoma who have received chemoradiotherapy (also referred to as a predictive marker of prognosis) is for discriminating patients with good prognosis and patients with poor prognosis, in a group of patients who underwent surgical resection of cancer after chemoradiotherapy.

"Chemoradiotherapy" as used herein may be "chemoradiotherapy" performed alone or "preoperative chemoradiotherapy" and "postoperative chemoradiotherapy" performed before and after surgery, respectively. Alternatively, the chemoradiotherapy may be combined with treatment other than surgery. Although a combination of "chemotherapy" using, for example, an anticancer drug and "radiotherapy" using radiation is preferable, the "chemoradiotherapy" may be either the chemotherapy performed alone or the radiotherapy performed alone. The anticancer drug used in the chemotherapy is not limited and any anticancer drug which is well-known to those skilled in the art can be used, such as fluorouracil or CDDP. Dose and schedule of administration of the anticancer drug depend on the type of the anticancer drug and conditions of the patient. Two or more anticancer drugs may be co-administered. Intensity and duration of radiation in the radiotherapy are not specifically limited as long as they fall within a range typically used for the treatment of cancer.

The term "cancer" as used herein means neoplasms such as carcinomas originating from epithelial cells, tumors originating from non-epithelial cells, and blood cancers, and is not limited to a specific cancer staging. The cancer for which the prognosis or the response to chemoradiotherapy is predicted is preferably squamous cell carcinoma, more preferably head and neck squamous cell carcinoma or esophageal squamous cell carcinoma, and most preferably esophageal squamous cell carcinoma. Examples of the head and neck squamous cell carcinoma include nasal cavity cancer, maxillary cancer, maxillary sinus cancer, tongue cancer, carcinoma of the mouth floor, gingival carcinoma, buccal mucosa cancer, nasopharyngeal carcinoma, oropharyngeal cancer, hypopharyngeal cancer, and laryngeal cancer. Examples of the esophageal squamous cell carcinoma include upper esophageal cancer, middle esophageal cancer, and lower esophageal cancer. Epithelium of oral mucosa and epithelium of esophageal mucosa are epithelial tissues of the same type from the developmental and histological viewpoint.

The predictive marker of response according to the present invention is a soluble interleukin-6 receptor (also referred to as sIL6R). By measuring the amount of a predictive marker of response in the blood obtained from a vertebrate animal suffering from a cancer before treatment with chemoradiotherapy, the response of the animal to the chemoradiotherapy for the cancer can be predicted.

The predictive marker of prognosis according to the present invention is the soluble interleukin-6 receptor (sIL6R). Thus, the soluble interleukin-6 receptor can be used effectively as the predictive marker of response as well as the predictive marker of prognosis.

### == Measurement of the biomarker ==

An animal for which the biomarker is to be measured may be a human or any vertebrate animal, as long as it has the biomarker according to the present invention. The animal is preferably a mammal such as a human, a mouse, a rat, a dog, a cat, a horse, a sheep, a rabbit, a pig, and a monkey. It is most preferable that the animal is a human. The age and sex of the vertebrate animal are not specifically limited. The following description is made for a human patient as an example.

The blood is preferably pretreated before being subjected to measurement of the biomarker. For example, the serum or plasma is preferably separated from the blood on standing or by centrifugation and the separated serum or plasma is used for the measurement.

As to the measurement of the biomarker according to the present invention, only the biomarker may be measured .The biomarker to be measured can be appropriately determined by those skilled in the art in consideration of, for example, a method of measurement and the amount of the blood. The amount of the biomarker according to the present invention may be determined at the same time as the measurement of the content or concentration of one or more other substances.

The amount of the biomarker in the drawn blood can be determined using a known method. For example, the amount of a biomarker may be determined using an antibody specific to that biomarker using a well-known method such as ELISA (enzyme-linked immunosorbent assay) including direct competitive ELISA, indirect competitive ELISA, and sandwich ELISA, RIA (radioimmunoassay), flowmetry, immunochromatography. In this case, the antibody specific to the biomarker may be polyclonal or monoclonal and is not limited by the animal species from which the antibody is derived. The antibody may be a full-length immunoglobulin or a partial antibody. The term "partial antibody" refers to a fragment of antibody with the antigen-binding site having antigen-binding activity. Examples of the partial antibody include a Fab fragment and a F(ab')₂ fragment. When the antibody is labeled with a label, examples of the label include, but not limited to, fluorescent substances (e.g., FITC, rhodamine, and phalloidine), colloidal particles such as gold, fluorescent microbeads such as Luminex (registered trademark, Luminex Corporation), heavy metals (e.g., gold and platinum), chromoproteins (e.g., phycoerythrin and phycocyanin), radioisotopes (e.g., ³H, ¹⁴C, ³²P, ³⁵S, ¹²⁵I and ¹³¹I), enzymes (e.g., peroxidase and alkaline phosphatase), biotin and streptavidin.

An example of the measurement performed using sandwich ELISA is given below. First, antibody (antibody 1) that is specific to a biomarker is immobilized on a solid phase such as a microplate. When the serum is added to the solid phase, the biomarker in the serum binds to the antibody, producing an immune complex. After the removal of excess serum, antibody (antibody 2) that recognizes an epitope different from the epitope recognized by the antibody 1 is added to the labeled biomarker, to allow the antibody 2 to bind to the biomarker. After the removal of excess antibody 2 by washing, the amount of the label remained on the microplate is measured. A calibration curve is made in advance, which represents a relationship between the amount of the marker added to the microplate and the amount of the remaining label. This calibration curve is used to calculate the amount of the marker in the blood.

Another example is given below for the measurement performed using fluorescent bead-based array system Luminex (Hitachi Software Engineering Co., Ltd.) which is an example of flowmetry. First, an antibody (antibody 1) that is specific to a biomarker is labeled with fluorescent microbeads. When this labeled antibody is mixed with the serum, the antibody binds to the biomarker in the serum, producing an immune complex. Then, a biomarker-specific antibody (antibody 2) labeled with biotin, which recognizes an epitope different from the epitope recognized by the biomarker-specific antibody 1, is added thereto. Then the antibody 2 binds to the biomarker that has been bound with the antibody 1. When an avidin-fluorescent dye is added, the dye binds to the biotin labeling the antibody 2, producing an avidin-biotin complex. This sample is subjected to flow cytometry, which specifies the fluorescent microbeads by the wavelength of the fluorescence. The amount of the biomarker is then quantified by the strength of the fluorescence of the surface of the specified beads. With this method, two or more biomarkers can be measured simultaneously by labeling each of the antibodies (antibodies 1) that is specific to each of different biomarkers with each of fluorescent dyes having different excitation wavelengths.

### == Use of the biomarker ==

Use of the biomarker according to the present invention includes, for example, following modes and aspects.

### <Prediction of Response to Chemoradiotherapy>

By measuring the amount of the biomarker in the blood obtained from a vertebrate animal having cancer before treatment with the chemoradiotherapy, the animal's response to the chemoradiotherapy for cancer can be predicted.

The amount of the biomarker is preferably represented as an absolute concentration of the biomarker. The amount is, however, not limited as long as it is related to the absolute concentration of the biomarker so that the absolute concentration can be compared among the individuals using it. The amount may be a relative concentration, merely a weight per a unit volume, or raw data measured to determine the absolute concentration.

sIL6Ris the predictive marker of response which are useful for the prediction of the efficacy of the chemoradiotherapy. For example, the amount of the biomarker in the blood is obtained in a certain group of patients before application of the chemoradiotherapy. Thereafter, their responses to the chemoradiotherapy are evaluated, the patients are divided into a responder group with a good therapeutic response and a non-responder group with a poor therapeutic response and the ranges of the amount of the biomarker in the blood are then determined for each of the groups. To divide the patients into the groups either "with a good therapeutic response" or "with a poor therapeutic response", a criteria predetermined by those skilled in the art in consideration with a well-known technique may be used. For example, for chemoradiotherapy for esophageal squamous cell carcinoma, the grade 3 effects specified by the histopathological criteria (Classification of Esophageal Cancer, 10th edition) may be defined as a good therapeutic response, and the grade 2 or lower effects may be defined as a poor therapeutic response. The amount of the biomarker in the blood of each patient to be diagnosed may be determined and then it may be determined which range the result falls in. When the result falls in the range of the responder group, the chemoradiotherapy may be applied. When the result falls in the range of the non-responder group, or does not fall in the range of the responder group, the chemoradiotherapy may not be applied.

Alternatively, a threshold, instead of the aforementioned ranges, may be determined for the amount of the biomarker in the blood obtained before treatment with the chemoradiotherapy to predict the response. A method to determine the threshold is not specifically limited and a routine method known to those skilled in the art can be used. The threshold may be determined such that a first predetermined percentage of patients who will respond is included below the threshold and a second predetermined percentage of patients who will not respond is included at or above the threshold. The threshold is preferably determined such that the first predetermined percentage and the second predetermined percentage are both high. The percentages are preferably 50% or higher, more preferably 70% or higher, yet further preferably 90% or higher, and most preferably 100%. Setting of higher percentages for both provides higher specificity and sensitivity. This means that the patients to be diagnosed can be discriminated into the responder and non-responder groups with high accuracy, by means of determining the threshold such that both the specificity and the sensitivity become high. These values are preferably 50% or higher, more preferably 70% or higher, yet further preferably 90% or higher, and most preferably 100%. Alternatively, the threshold may be determined so that the best chi-square value for the discrimination is obtained by using a statistical software such as JMP available from SAS Institute Japan. More specifically, for example, the threshold of blood sIL6R concentration may be set from 10 to 35 ng/ml and preferably from 20 to 30 ng/ml.

More specifically, for example, the threshold of blood sIL6R concentration may be set from 10 to 35 ng/ml, and the patients whose value is smaller than the threshold may be considered as the responders and the patients whose value is equal to or larger than the threshold may be considered as the non-responders. The threshold of blood concentration is, however, preferably set from 20 to 30 ng/ml, and most preferably set at 30 ng/ml.

In view of the accuracy of prediction of the therapeutic response, the animal from which the blood is obtained to determine a range or a threshold for the amount of the marker and the animal to be diagnosed preferably belong to the same species and suffer from the same type of cancer.

The biomarker according to the present invention is SIL6R. The prediction of the therapeutic response using the predictive marker of response according to the present invention may be combined with other diagnostic method for cancer. For the purpose of convenience, the prediction is preferably combined with other blood markers.

### <Prediction of prognosis after treatment>

By measuring the amount of the biomarker in the blood obtained from a vertebrate animal having cancer before chemoradiotherapy, the prognosis of the animal after surgical resection of the cancer can be predicted.

Prognosis of cancer patients is associated with years of survival from the beginning of treatment. For humans, prognosis may be considered good when a patient survives for 5 years from the beginning of treatment and prognosis may be considered poor when the patient dies before 5 years from the beginning of treatment.

It is noted that sIL6R is a predictive marker of prognosis with which patients with a longer prognosis and patients with a shorter prognosis can be discriminated efficiently. A range or a threshold of the blood concentration of the predictive marker of prognosis may be determined in a manner similar to the one described in the "Prediction of response to chemoradiotherapy", and prognosis may be predicted based on the range or the threshold.

### Examples

[Example 1] This example shows that response to chemoradiotherapy for cancer tissue is associated with a survival rate of a patient.

At the Third Department of Surgery, Tokyo Medical University Hospital, 37 patients with advanced esophageal squamous cell carcinoma were treated with preoperative chemoradiotherapy, and esophagectomy was performed 4 weeks after the chemoradiotherapy. For the chemotherapy, fluorouracil and CDDP were used. For the radiotherapy, an electron beam from Linac (linear accelerator) was used.

The chemoradiotherapy continued for 4 weeks, and performed for the first five consecutive days for each week. The patients were administered with 350 mg/m² (patient's body surface area) of fluorouracil (5-FU, Kyowa Hakko Kirin Co., Ltd.) and 5 mg/m² (patient's body surface area) of CDDP (Nippon Kayaku Co., Ltd.) daily, and 7000 mg/m² and 100 mg/m² in total, respectively, for a total treatment period. In addition, the radiotherapy was given at 2 Gy daily fractions to a total dose of 40 Gy over the same total treatment period.

Histopathologic diagnosis of the tissue resected during the esophagectomy was performed to determine a preoperative response to the chemoradiotherapy according to the histopathological criteria (Classification of Esophageal Cancer, 10th edition, see Table 1). In addition, the aforementioned 37 patients with esophageal squamous cell carcinoma were followed up for up to 9 years from the beginning of the treatment.

**Table 1**

| Grade | | Effects | Tissue Condition |
|---|---|---|---|
| 0 | | ineffective | no effect on cancer tissue and cancer cells |
| 1 | 1a | slightly effective | cancer cells appeared to be grown account for 2/3 or more of cancer tissues |
| | 1b | | cancer cells appeared to be grown account for 1/3 or more but less than 2/3 of cancer tissue |
| 2 | | moderately effective | cancer cells appeared to be grown account for less than 1/3 of cancer tissue |
| 3 | | markedly effective | no cancer cells appeared to be grown |

The 37 patients with esophageal squamous cell carcinoma were classified into two groups: a group of patients with the grade 3 effects to the chemoradiotherapy in the histopathologic diagnosis and a group of patients with the grade 1 or 2 effects. Table 2 below shows the gender, age, tumor location, and clinical stage of each group.

**Table 2**

| | Grade 3 | Grade 1 + 2 |
|---|---|---|
| | n = 7 | n = 30(12 + 18) |
| Age (mean + S.D.) | 64.3 ± 7.16 | 60.6 ± 7.59 |
| Gender (%) | | |
| Male | 5 (71.4) | 27 (90) |
| Female | 2 (28.6) | 3 (10) |
| Tumor location (%) | | |
| Ce | 0 | 3 (10) |
| Te | 6 (85.7) | 27 (90) |
| Ut | 1 (16.7) | 5 (18.5) |
| Mt | 2 (33.3) | 15 (55.6) |
| Lt | 3 (50) | 7 (25.9) |
| Ae | 1 (14.3) | 0 |
| Clinical stage (%) | | |
| II | 0 | 4 (13.3) |
| III | 7 (100) | 20 (66.7) |
| IV | 0 | 6 (20) |

| | | |
|---|---|---|
| (For the "tumor location" and the "clinical stage" in this table, see, Hayashida Y, Honda K, Osaka Y, Hara T, Umaki T, Tsuchida A, Aoki T, Hirohashi S, Yamada T. Possible prediction of chemoradiosensitivity of esophageal cancer by serum protein profiling. Clin. Cancer Res. 2005 Nov 15; 11(22): 8042-7.) | | |

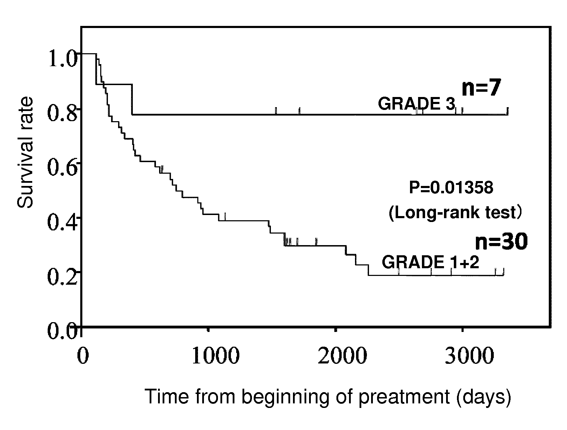

As shown in Table 2, 7 patients were classified as grade 3 and 30 patients were classified as grade 1 or 2 in the histopathologic diagnosis after the chemoradiotherapy. Fig. 1 shows a graph of survival rates of these patients for up to 9 years.

As shown in Fig. 1, the 9-year survival rate of the patients with the grade 3 effects of the chemoradiotherapy was about 80%, while the 9-year survival rate of the patients with the grade 1 or 2 effects was about 20%. In this way, the response to chemoradiotherapy is associated with the survival rate of the patients.
[Example 2] This example shows that the response to chemoradiotherapy can be predicted by using the biomarkers sIL6R, MIP-1 and PAI-1.

The biomarker sIL6R was measured using a fluorescent bead-based array system Luminex (Hitachi Software Engineering Co., Ltd.) or sandwich ELISA on the blood obtained from the aforementioned patients with esophageal squamous cell carcinoma before the chemoradiotherapy.

### == Measurement by fluorescent bead-based array system luminex ==

Extracelular Luminex Kit sIL6R (catalog No. LHR0061) available from Biosource International Inc., was used for the measurement of sIL6R. The analysis were contracted out to Hitachi Software Engineering Co., Ltd.

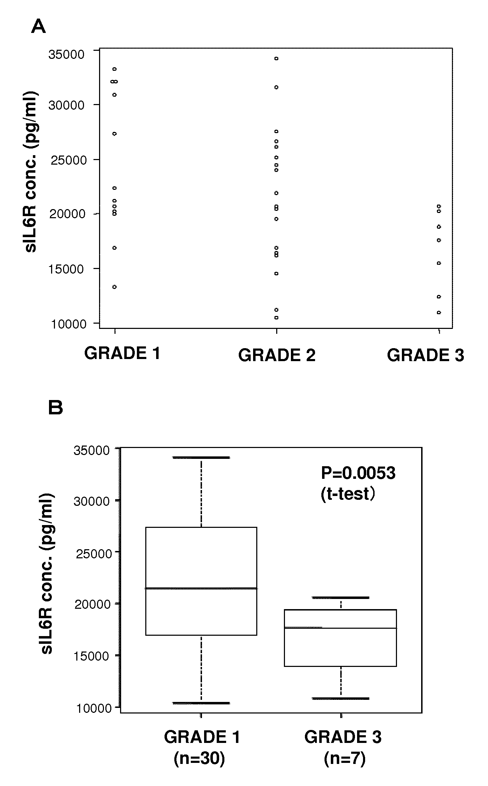

Figs. 2A and 2B show the concentration of sIL6R for the patients of the grades 1, 2, and 3. The concentration of sIL6R was significantly lower in the group of the grade 3 patients compared to the group of the grade 1 + 2 patients.

### == Measurement by sandwich ELISA ==

Blood sIL6R concentration was measured in SRL on a contract basis by sandwich ELISA using Quantikine Human IL-6 sR Immunoassay (R&D Systems).

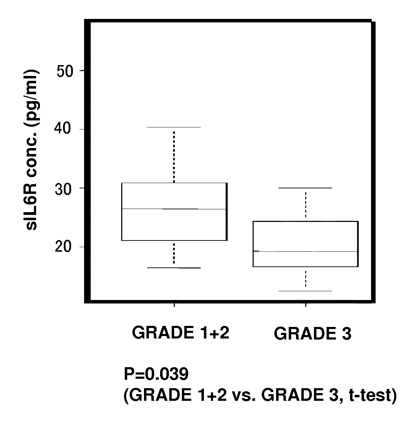

As shown in Fig. 3, measurements by the sandwich ELISA showed that the concentration of sIL6R was significantly lower in the responder group (grade 3) compared to the non-responder group (grades 1, 2).

Thus, the blood concentration of sIL6R, are significantly different between the group (grade 3) with a good response to the chemoradiotherapy and the group (grades 1, 2) with a poor response to the chemoradiotherapy. Thus, this biomarker can be used to predict the response to the chemoradiotherapy.

However, the distribution of the concentration of sIL6R in the responder group overlapped with the distribution in the non-responder group. Therefore, this marker is useful to identify an individual that will not respond to the chemoradiotherapy. For example, the highest concentration of sIL6R in the responder group is used as a threshold. Then the individual that will not respond can be distinguished effectively by identifying the individual whose value of concentration is larger than that threshold is identified. Since the individual whose value of concentration is smaller than the threshold is more likely to respond to the chemoradiotherapy, the chemoradiotherapy may be applied. It is, however, preferable that a decision is made depending on the situation.
[Example 3] This example shows that prognosis of a patient can be predicted using the biomarker according to the present invention.

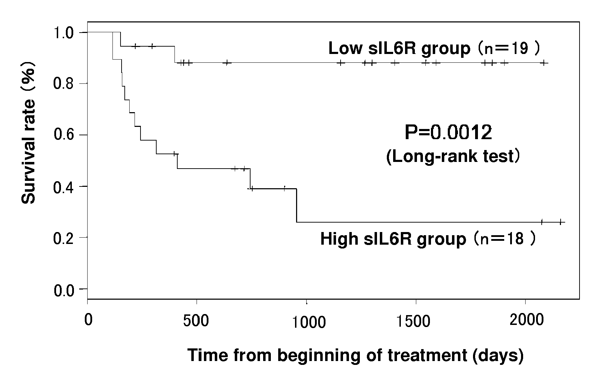

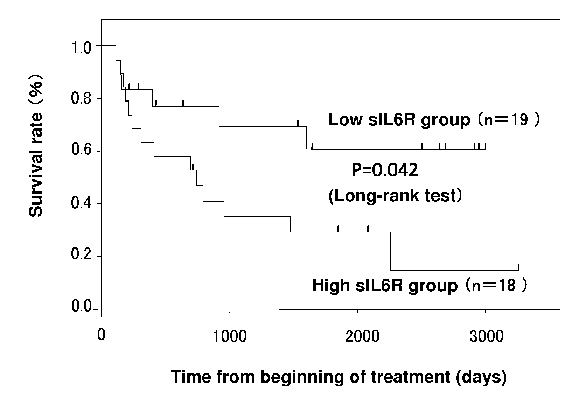

For the concentration of sIL6R measured by the fluorescent bead-based array system or the sandwich ELISA in Example 2, 30 ng/ml was set as a threshold. Figs. 4 and 5 show graphs of survival rates for up to 6 and 9 years, respectively, from the beginning of the treatment in a high sIL6R group with a value of the blood concentration larger than the threshold and a low sIL6R group with a value of the blood concentration smaller than the threshold.

The measurement by the fluorescent bead-based array system indicated that 18 cases belong to the high sIL6R group and 19 cases belong to the low sIL6R group. The 6-year survival rate of about 90% in the low sIL6R group was significantly higher compared to that of about 25% in the high sIL6R group (P = 0.0012, long-rank study). The sensitivity and the specificity as a marker of response after 6 years are 77% and 89%, respectively.

The measurement by the sandwich ELISA also indicated that 18 cases belong to the high sIL6R group and 19 cases belong to the low sIL6R group. The 9-year survival rate of about 60% in the low sIL6R group was significantly higher compared to that of about 15% in the high sIL6R group (P = 0.042, long-rank study). The sensitivity and the specificity as a marker of response after 9 years are 78% and 57%, respectively.

Thus, the prognosis of a patient can be predicted using the biomarker sIL6R according to the present invention.
[Example 4] This example shows that sIL6R reflects only the response to chemoradiotherapy in patients with esophageal squamous cell carcinoma and the prognosis of such patients, and does not reflect other factors.

The Cox proportional hazards regression model was used to determine whether the age, gender, tumor location, and clinical stage were associated with the blood SIL6R concentration in the patients with esophageal squamous cell carcinoma shown in Table 2. Results are given in Tables 3 and 4.

**Table 3**

| Univariate Analysis | n | Relative Risk | 95% C.I. | p value |
|---|---|---|---|---|
| Age (<65/65≤) | 25/12 | 1.308 | 0.5765-2.9679 | 0.520666 |
| Gender (male/female) | 32/5 | 0.3113 | 0.0729-1.3300 | 0.115234 |
| Tumor Location (Ce,Ut/Mt,Lt,Ae) | 9/28 | 0.6962 | 0.2593-1.8695 | 0.472447 |
| clinical stage (0, I, II/III, IV) | 19/18 | 3.0647 | 1.3369-7.0255 | 0.008145 |
| sIL6R (median) | 18/19 | 3.1881 | 1.3494-7.5322 | 0.008214 |

**Table 4**

| Multivariate Analysis | n | Relative Risk | 95% C.I. | p value |
|---|---|---|---|---|
| Clinical Stage (0, I, II/III, IV) | 19/18 | 2.4983 | 1.1016-6.5221 | 0.033545 |
| sIL6R (median) | 18/19 | 2.8715 | 1.2033-6.8527 | 0.017456 |

These results indicate that sIL6R is independent of the age, gender, and tumor location of the patients and is associated with the clinical stage.

### Industrial applicability

The present invention provides a biomarker and a method of measuring the same for determining whether or not chemoradiotherapy is applicable to a patient with cancer.

## Claims

1. Use of a soluble interleukin-6 receptor for predicting response to chemoradiotherapy for a squamous cell carcinoma.

2. Use of a soluble interleukin-6 receptor for predicting prognosis of a patient with a squamous cell carcinoma, wherein the patient has received chemoradiotherapy.

3. The use according to Claim 1 or 2, wherein the squamous cell carcinoma is a head and neck squamous cell carcinoma or an esophageal squamous cell carcinoma.

4. The use according to Claim 2, wherein the chemoradiotherapy is preoperative chemoradiotherapy.

5. A method of predicting response to chemoradiotherapy for a squamous cell carcinoma, comprising:
measuring the concentration of a soluble interleukin-6 receptor in blood obtained before treatment with chemoradiotherapy.

6. A method of predicting prognosis after treatment with chemoradiotherapy, of a patient with squamous cell carcinoma after surgical resection of squamous cell carcinoma, comprising:
measuring the concentration of a soluble interleukin-6 receptor in blood obtained from the patient before treatment with chemoradiotherapy.

7. The method according to Claim 5 or 6, wherein the concentration of the biomarker is measured using an antibody specific to the soluble interleukin-6 receptor.

8. The method according to any one of Claims 5-7, wherein the squamous cell carcinoma is a head and neck squamous cell carcinoma or an esophageal squamous cell carcinoma.

## Patentansprüche

1. Gebrauch eines löslichen Interleukin-6-Rezeptors zum Vorhersagen einer Reaktion auf Chemo-Strahlentherapie für ein Plattenepithelkarzinom.

2. Gebrauch eines löslichen Interleukin-6-Rezeptors zum Vorhersagen einer Prognose eines Patienten mit einem Plattenepithelkarzinom, wobei der Patient Chemo-Strahlentherapie erhalten hat.

3. Gebrauch nach Anspruch 1 oder 2, wobei es sich bei dem Plattenepithelkarzinom um ein Plattenepithelkarzinom von Kopf und Hals oder ein Plattenepithelkarzinom des Ösophagus handelt.

4. Gebrauch nach Anspruch 2, wobei es sich bei der Chemo-Strahlentherapie um präoperative Chemo-Strahlentherapie handelt.

5. Verfahren zum Vorhersagen einer Reaktion auf Chemo-Strahlentherapie für ein Plattenepithelkarzinom, Folgendes umfassend:
Messen der Konzentration eines löslichen Interleukin-6-Rezeptors im Blut, das vor dem Behandeln mit Chemo-Strahlentherapie gewonnen wurde.

6. Verfahren zum Vorhersagen einer Prognose nach dem Behandeln eines Patienten mit Plattenepithelkarzinom mit Chemo-Strahlentherapie nach chirurgischer Resektion eines Plattenepithelkarzinoms, Folgendes umfassend:
Messen der Konzentration eines löslichen Interleukin-6-Rezeptors im Blut, das vor dem Behandeln mit Chemo-Strahlentherapie von dem Patienten gewonnen wurde.

7. Verfahren nach Anspruch 5 oder 6, wobei die Konzentration des Biomarkers unter Verwendung eines für den löslichen Interleukin-6-Rezeptor spezifischen Antikörpers gemessen wird.

8. Verfahren nach einem der Ansprüche 5-7, wobei es sich bei dem Plattenepithelkarzinom um ein Plattenepithelkarzinom von Kopf und Hals oder ein Plattenepithelkarzinom des Ösophagus handelt.

## Revendications

1. Utilisation d'un récepteur soluble de l'interleukine-6 pour prédire une réponse à une radiochimiothérapie pour un épithélioma spinocellulaire.

2. Utilisation d'un récepteur soluble de l'interleukine-6 pour prédire le pronostic d'un patient atteint d'un épithélioma spinocellulaire, le patient ayant reçu une radiochimiothérapie.

3. Utilisation selon la revendication 1 ou 2, l'épithélioma spinocellulaire étant un épithélioma spinocellulaire de la tête et du cou ou un épithélioma spinocellulaire de l'oesophage.

4. Utilisation selon la revendication 2, la radiochimiothérapie étant une radiochimiothérapie préopératoire.

5. Procédé de prédiction d'une réponse à une radiochimiothérapie pour un épithélioma spinocellulaire, comprenant :
la mesure de la concentration d'un récepteur soluble de l'interleukine-6 dans le sang obtenue avant un traitement par radiochimiothérapie.

6. Procédé de prédiction d'un pronostic après un traitement par radiochimiothérapie d'un patient atteint d'un épithélioma spinocellulaire après une résection chirurgicale d'un épithélioma spinocellulaire, comprenant :
la mesure de la concentration d'un récepteur soluble de l'interleukine-6 dans le sang obtenue auprès du patient avant un traitement par radiochimiothérapie.

7. Procédé selon la revendication 5 ou 6, la concentration du biomarqueur étant mesurée à l'aide d'un anticorps spécifique au récepteur soluble de l'interleukine-6.

8. Procédé selon l'une quelconque des revendications 5-7, l'épithélioma spinocellulaire étant un épithélioma spinocellulaire de la tête et du cou ou un épithélioma spinocellulaire de l'oesophage.
